# EUROPÄISCHE PATENTSCHRIFT

(11) **EP 0 885 969 B1**
(45) Veröffentlichungstag und Bekanntmachung des Hinweises auf die Patenterteilung: **29.08.2001**
(21) Anmeldenummer: 98110716.2
(22) Anmeldetag: 10.06.1998
(51) Int. Cl.: C12Q 1/04, C12Q 1/14, C12N 1/20

(54) **Nährboden zum Nachweis Streptococcus mutans**
Culture medium to detect Streptococcus mutans
Milieu à culture pour détecter le Streptococcus mutans

(30) Priorität: 13.06.1997 DE 19724970
(43) Veröffentlichungstag der Anmeldung: 23.12.1998
(73) Patentinhaber: Laurisch, Lutz, Dr., 41352 Korschenbroich (DE)
(72) Erfinder: Laurisch, Lutz, Dr., 41352 Korschenbroich (DE)
(74) Vertreter: Lau-Loskill, Philipp, Dipl.-Phys.

(56) Entgegenhaltungen:
- EP-A- 0 190 893
- US-A- 3 890 200
- US-A- 4 468 456
- PATENT ABSTRACTS OF JAPAN vol. 9, no. 145 (C-287), 20. Juni 1985 & JP 60 027389 A (T. KENICHI ET AL.)

## Beschreibung

Die vorliegende Erfindung betrifft einen Nährboden zum Nachweis von Mutans Streptokokken im Speichel oder im Plaque mit den Merkmalen des Oberbegriffs des Patentanspruchs 1 sowie ein Analysenset mit den Merkmalen des Oberbegriffs des Patentanspruchs 20.

Es ist bekannt, im Bereich der prophylaktischen Zahnheilkunde im Speichel oder im Plaque der Zähne die Anwesenheit von Mutans Streptokokken (streptococcus mutans) zu untersuchen und deren Konzentration quantitativ zu bestimmen. Um einen derartigen qualitativen und quantitativen Nachweis zu führen, wird eine vorgegebene Speichelmenge oder eine von den Zähnen abgekratzte Menge eines Plaques auf einen Nährboden aufgetragen, wobei hierfür der an sich bekannten Mitis Salivarius Agars häufig zur Anwendung gelangt.

So beschreibt beispielsweise die US 3,890,200 B ein selektives Medium zum Nachweis von Mutans Streptokokken, das als Nährboden einen Mitis Salivarius Agar enthält, dem Zucker bis zu einer Höchstkonzentration von 200 g pro 1.000 ml anwendungsfertigem Agar zugesetzt ist. Hieraus errechnet sich ein Verhältnis von Agar zu Zucker von etwa 1:0,2. Ausdrücklich stellt die US 3,890,200 B heraus, daß höhere Zuckerkonzentrationen erhebliche negative Einflüsse auf das Wachstum und die Morphologie von aus Speichel isolierten Mutans Streptokokken haben.

Auch die EP 0 190 893 A beschreibt ein Nachweisverfahren für Mutans Streptokokken, wobei hierfür ein auf einen inerten Träger aufgetragener Nährboden verwendet wird. Dieser bekannte Nährboden auf der Basis eines Mitis Salivarius Agar weist zusätzlich 150 g Saccharose sowie Bacitracin auf, so daß sich hieraus ein Massenverhältnis von Agar zu Zucker von ebenfalls 1:1,66 errechnet.

Desweiteren ist es zur Erstellung einer Diagnostik, insbesondere zur Einschätzung des Risikos der Kariesentstehung, erforderlich, die Konzentration an Lactobazillen im Speichel und/oder Plaque zu bestimmen, wobei hierfür ebenfalls ein Nährboden verwendet wird. Auf diesen Nährboden auf der Basis eines Rogosa Agars wird ebenso, wie zuvor für den Nachweis von Mutans Streptokokken beschrieben, eine Probe des Speichels bzw. des Plaques aufgetragen, so daß nach einer entsprechenden Bebrütungszeit dann visuell der bebrütete Boden qualitativ und quantitativ ausgewertet werden kann. Der hierfür eingesetzte Nährboden auf der Basis eines herkömmlichen Rogosa Agars weist eine Zusammensetzung auf, wie diese nachfolgend angegeben ist.

| | |
|---|---|
| Agar Agar | 19 g |
| Tryptone | 10 g |
| Hefeextrakt | 5 g |
| Glucose | 20 g |
| Kaliumhydrogenphosphat | 6 g |
| Ammoniumhydrogencitrat | 2 g |
| Mangan-II-Sulfat 1-Hydrat | 0,1 g |
| Eisen-II-Sulfat 7-Hydrat | 0,03 g |
| Tween 80 | 1 g |

Zur Herstellung des anwendungsfertigen Rogosa-Nährbodens werden die zuvor angegebenen Bestandteile in 1 l Wasser gegeben.

Darüber hinaus ist es zur Erstellung einer einwandfreien Diagnostik, insbesondere zur Festlegung von konkreten Maßnahmen zur Beseitigung des Risikos einer Kariesentstehung, erforderlich, die Pufferkapazität des Speichels zu messen. Hierbei wird bei den heute verfügbaren Analysensets diese Pufferkapazität des Speichels dadurch ermittelt, daß ein mit einem Indikator getränkter Teststreifen mit Speichel betropft wird, wobei nach einer Einwirkzeit von etwa zwei Minuten dann der erforderliche Farbumschlag des Teststreifens visuell ermittelt wird. Hierbei läßt jedoch der hierfür verwendete Teststreifen nur eine grobe Messung der als pH-Wert definierten Pufferkapazität zu, so daß die Aussage der Messung der Pufferkapazität unsicher und von der Geschicklichkeit des jeweiligen Anwenders abhängig ist.

Der vorliegenden Erfindung liegt die Aufgabe zugrunde, einen Nährboden der angegebenen Art zur Verfügung zu stellen, der eine bessere Aussage bezüglich der Konzentration an Streptokokken mutans ermöglicht.

Diese Aufgabe wird durch einen Nährboden mit den kennzeichnenden Merkmalen des Patentanspruchs 1 gelöst.

Der erfindungsgemäße Nährboden zum Nachweis von Mutans Streptokokken (streptococcus mutans) im Speichel und/oder im Plaque sieht vor, daß der erfindungsgemäße Nährboden neben einem Agar zusätzlich noch mindestens einen Zucker enthält, wobei das Massenverhältnis von dem Agar zu dem mindestens einen Zucker zwischen 1:3,89 und 1:12 variiert. Mit anderen Worten unterscheidet sich somit der erfindungsgemäße Nährboden von dem an sich bekannten Nährböden dadurch, daß der erfindungsgemäße Nährboden zusätzlich noch den mindestens einen Zucker in dem zuvor angegebenen Massenverhältnis aufweist.

Überraschend wurde festgestellt, daß der erfindungsgemäße Nährboden eine hohe Selektivität bezüglich der Mutans Streptokokken besitzt, wobei diese hohe Selektivität für Mutans Streptokokken darauf zurückgeführt wird, daß der erfindungsgemäße Nährboden im Vergleich zur herkömmlichen und im Handel befindlichen Nährböden zusätzlich noch eine erhebliche Menge Zucker aufweist. Diese verbesserte Selektivität des erfindungsgemäßen Nährbodens drückt sich im Vergleich zu herkömmlichen Nährböden dadurch aus, daß der erfindungsgemäße Nährboden eine signifikant höhere Keimausbeute an Mutans Streptokokken besitzt, was anhand von 135 Speichelisolaten von 55 Kindern bestätigt wurde. Auch behindert dieser zusätzliche Zuckerzusatz die Zubereitung eines anwendungsfertigen Nährbodens aus dem erfindungsgemäßen Nährboden nicht, wobei für die einfache und schnelle Zubereitung der erfindungsgemäße trockene Nährboden lediglich in der erforderlichen Menge Wasser aufgenommen wird.

Auch läßt sich der erfindungsgemäße Nährboden besonders einfach herstellen, da es hierfür lediglich erforderlich ist, einen geeigneten Agar mit der erforderlichen Zuckermenge zu vermischen, wobei die Vermischung des Agars mit dem Zucker sowohl bei der Herstellung eines anwendungsfertigen Nährbodens, d.h. bei der Aufnahme des Agars und des Zuckers in Wasser, als auch durch Vermischung des trockenen Agars mit dem mindestens einen Zucker erfolgen kann.

Eine erste Ausführungsform des erfindungsgemäßen Nährbodens sieht vor, daß hierbei das Massenverhältnis zwischen dem Agar und dem mindestens einen Zucker zwischen 1:3,89 und 1:4,25 liegt. Hier hat sich gezeigt, daß sich diese bevorzugte Ausführungsform besonders einfach und schnell in Wasser aufnehmen läßt, so daß dementsprechend schnell und unproblematisch ein anwendungsfertiger Nährboden erstellt werden kann.

Eine weitere Ausführungsform des erfindungsgemäßen Bodens, die eine extrem hohe Selektivität zum Nachweis der Mutans Streptokokken aufweist, sieht vor, daß hierbei das Massenverhältnis zwischen dem Agar und dem mindestens einen Zucker zwischen 1:5,1 und 1:10 variiert. Obwohl diese Ausführungsform des erfindungsgemäßen Nährbodens eine extrem hohe Zuckerkonzentration aufweist, konnte überraschend festgestellt werden, daß auf einem derartig mit Zucker versetzten Nährboden die Mutans Streptokokken, die aus dem menschlichen Speichel oder aus dem Plaque gewonnen wurden, hervorragend und selektiv wuchsen.

Vorstehend und nachfolgend wird wiederholt der Begriff Agar verwendet, wobei hierunter insbesondere der aus roten Meeresalgen gewonnene und getrocknete Schleim sowie hochmolekulare Galaktoside, insbesondere Salze von Galaktose-Schwefelsäureestern, verstanden wird, sofern nachstehend nicht spezielle Agar genannt sind.

Bezüglich des in dem erfindungsgemäßen Nährboden enthaltenen mindestens einen Zuckers ist festzuhalten, daß es sich bei diesem Zucker um ein Monosaccharid und/oder ein Monosaccharid-Derivat handeln kann. Vorzugsweise weist jedoch der erfindungsgemäße Nährboden als Zucker ein Disaccharide, insbesondere Saccharose und/oder ein Saccharose-Derivat, auf.

Abhängig von dem jeweiligen Anwendungsgebiet besteht im einfachsten Fall der erfindungsgemäße Nährboden lediglich aus dem herkömmlichen Agar sowie dem zuvor erläuterten Zucker in den dort genannten Massenverhältnissen. Wird eine derartige Ausführungsform des erfindungsgemäßen Nährbodens dann von geschultem Fachpersonal angewendet, so beispielsweise in entsprechenden Kliniken, ist es lediglich erforderlich, den erfindungsgemäßen Nährboden in einer entsprechenden Menge Wasser aufzunehmen, so daß dann entsprechende Schalen, beispielsweise Petrischalen, mit diesem anwendungsfertigen gelartigen Nährboden gefüllt werden können. Um bei dieser Ausführungsform des erfindungsgemäßen Nährbodens dann nach dem Inkubieren mit dem Speichel bzw. Plaque und der anschließenden Bebrütung eine sichere und reproduzierbare Unterscheidung zwischen dem aus dem Speichel oder dem Plaque angezüchteten Mutans Streptokokken und den möglicherweise mit angezüchteten Streptokokken, insbesondere den streptokokken parasanguis und streptokokken salivarius, herbeizuführen, empfiehlt es sich, eine bei mikrobiologischen Untersuchungen übliche Verdünnungsreihe durchzuführen. Hierbei wird der Speichel bzw. der Plaque auf ein Zehntel bis ein Tausendstel seiner ursprünglichen Konzentration mit einer physiologischen Kochsalzlösung verdünnt, was dazu führt, daß das Wachstum der zuvor genannten Streptokokken sowie von Lactobazillen, Aktinomyzeten, Staphylokokken oder gramnegative Bakterien eindeutig und wirksam unterdrückt wird. Dies wiederum bewirkt, daß besonders sicher und reproduzierbar die Mutans Streptokokken nachgewiesen und visuell quantifiziert werden können.

Eine besonders einfacher und sicherer Nachweis von Mutans Streptokokken, bei der auf die zuvor angesprochene Verdünnungsreihe ohne Verfälschung des Ergebnisses verzichtet werden kann, sieht vor, daß hierbei ein erfindungsgemäßer Nährboden zur Anwendung gelangt, der neben dem Agar und dem mindestens einen Zucker zusätzlich noch mindestens ein Peptidantibiotika-Komplex enthält, wobei die Konzentration dieses Peptidantibiotika-Komplexes zwischen 0,001 Gew.% und 0,0005 Gew.%, bezogen auf das Gesamtgewicht des Agar und des zugesetzten Zuckers, variiert.

Insbesondere weist die zuvor beschriebene Ausführungsform des erfindungsgemäßen Nährbodens als Peptidantibiotika-Komplex das im Handel erhältliche Bacitracin auf. Um aus dieser Ausführungsform des erfindungsgemäßen Nährbodens einen anwendungsfertigen Nährboden herzustellen, ist es lediglich erforderlich, das mit dem mindestens einen Zucker und dem Bacitracin versetzte Agar in der erforderlichen Menge Wasser aufzunehmen. Insbesondere konnte festgestellt werden, daß ein derartiger anwendungsfertiger erfindungsgemäßer Nährboden dann wirksam das Wachstum einer Mischflora, insbesondere das Wachstum von Streptokokken salivarius und/oder Streptokokken parasanguis, unterdrückt, ohne daß hierbei statistisch signifikant die Ausbeute an Mutans Streptokokken verringert wird. Somit ermöglicht diese Ausführungsform des erfindungsgemäßen Nährbodens, daß bei solchen Speichel- oder Plaqueproben, die neben Mutans Streptokokken noch andere Streptokokkenstämme enthalten, wirksam und einfach zwischen den Mutans Streptokokken und den anderen Streptokokken oder den zuvor genannten anderen Mikroorganismen unterschieden werden kann.

Ein besonders geeigneter Agar, der insbesondere bei dem erfindungsgemäßen Nährboden zur Anwendung gelangt, ist der bereits eingangs erwähnte Mitis Salivarius Agar.

Hierbei weist ein typischer Mitis Salivarius Agar, wie er beispielsweise im Handel erhältlich und von der Firma DIFCO, Augsburg, vertrieben wird, die nachfolgende Zusammensetzung auf:

| | |
|---|---|
| Bacto tryptose | 8 g bis 12 g, insbesondere 10 g |
| Proteose peptone Nr. 3 | 3 g bis 7 g, insbesondere 5 g |
| Proteose peptone | 3 g bis 7 g, insbesondere 5 g |
| Bacto dextrose | 0,5 g bis 1,5 g, insbesondere 1 g |
| Bacto saccharose | 30 g bis 60 g, insbesondere 50 g |
| Kaliumhydrogenphosphat | 3 g bis 5 g, insbesondere 4 g |
| Farbstoff, insbesondere Trypan blau | 0,05 g bis 0,09 g, insbesondere 0,075 g |
| Farbstoff, insbesondere Bacto kristall violett | 0,0006 g - 0,0001 g, insbesondere 0,008 g |
| Bacto Agar | 13 g bis 17 g, insbesondere 15 g |

Die zuvor aufgeführten Bestandteile werden zur Herstellung eines anwendungsfertigen Nährbodens in 1 l Wasser eingerührt.

Um insbesondere die Anwendung des erfindungsgemäßen Nährbodens in Zahnarztpraxen zu erleichtern, sieht eine weitere Ausführungsform des erfindungsgemäßen Nährbodens vor, daß der Nährboden einseitig auf einen inerten Träger aufgetragen ist. Hierbei ist es dann lediglich für den Nachweis von Mutans Streptokokken im Speichel erforderlich, den entsprechend steril verpackten und auf einen inerten Träger, insbesondere auf einen Kunststoffträger, aufgetragenen Nährboden aus der sterilen Verpackung zu entnehmen und diesen dann mit der zu untersuchenden Speichel- oder Plaqueprobe zu beaufschlagen, so daß nach einer entsprechenden sterilen Bebrütung die jeweilige Konzentration an Mutans Streptokokken abgelesen werden kann.

Wie bereits vorstehend wiederholt ausgeführt ist, läßt sich aus dem erfindungsgemäßen Nährboden leicht und schnell ein anwendungsfertiger erfindungsgemäßer Nährboden herstellen, wobei ein derartiger anwendungsfertiger Nährboden bevorzugt eine gelartige Konsistenz aufweist. Hierbei wird zur Erstellung der gelartigen Konsistenz der trockene erfindungsgemäße Nährboden, der den mit dem mindestens einen Zucker und ggf. mit dem Peptidantibiotika-Komplex, vorzugsweise mit dem Bacitracin, versetzte Agar, vorzugsweise den Mitis Salivarius Agar, aufweist, lediglich in Wasser aufgenommen, wobei die gewünschte gelartige Konsistenz dann durch die Menge des zugesetzten Wassers einstellbar ist.

Bevorzugt weist eine derartige gelartige Weiterbildung des anwendungsfertigen Nährbodens zwischen 70 g und 150 g des Agars, insbesondere des Mitis Salivarius Agars, zwischen 272,3 g und 1.800 g des mindestens einen Zuckers und so viel Wasser auf, um die erforderliche gelartige Konsistenz zu erstellen. Soll zusätzlich zu diesem gelartigen und anwendungsfertigen Nährboden dann noch der zuvor beschriebene Peptidantibiotika-Komplex, vorzugsweise das Bacitracin, zugesetzt werden, so variiert die Menge dieses Peptidantibiotika-Komplexes, vorzugsweise die Menge des Bacitracins, zwischen 1,4 mg und 12 mg, jeweils bezogen auf die Gesamtmenge des mindestens einen Zuckers und des Agars, insbesondere des Mitis Salivarius Agars.

Um bei dem zuvor beschriebenen und bei den nachfolgenden noch beschriebenen Ausführungsvarianten des erfindungsgemäßen Nährbodens, bei denen der erfindungsgemäße Nährboden auf einen inerten Träger aufgetragen ist, die Ablesegenauigkeit der angezüchteten Mutans Streptokokken zu erleichtern, sieht eine weitere Ausgestaltung des erfindungsgemäßen Nährbodens vor, daß hierbei der inerte Träger dunkel eingefärbt, insbesondere schwarz gefärbt, ist oder aus einem entsprechend farblich gestalteten Kunststoffmaterial besteht.

Eine andere Ausführungsform des erfindungsgemäßen Nährbodens, die gleichzeitig zur Bestimmung von Mutans Streptokokken und Lactobazillen oder von Hefepilzen geeignet ist, sieht vor, daß der erfindungsgemäße Nährboden, der neben dem Agar, insbesondere dem Mitis Salivarius Agar, desweiteren den mindestens einen Zucker in dem zuvor genannten Massenverhältnis und ggf. dem Peptidantibiotika-Komplex enthält, auf der einen Seite eines inerten Trägers, insbesondere auf der einen Seite des inerten Kunststoffträgers, aufgetragen ist, während die andere Seite des inerten Trägers mit einem weiteren Nährboden zum Nachweis von Lactobazillen im Speichel und/oder im Plaque oder mit einem weiteren Nährboden zum Nachweis von Hefepilzen im Speichel und/oder im Plaque versehen ist. Hierbei erlaubt eine derartige Ausführungsform des erfindungsgemäßen Nährbodens, daß mit einem einzigen Test gleichzeitig die für die Kariesprophylaxe erforderliche Konzentration an Mutans Streptokokken und Lactobazillen oder an Mutans Streptokokken und Hefepilzen im Speichel und/oder im Plaque bestimmt werden kann, so daß sich eine derartige Weiterbildung des erfindungsgemäßen Nährbodens insbesondere auch für die schnelle und sichere Diagnostik in Zahnarztpraxen verwenden läßt.

Vorzugsweise wird bei der zuvor beschriebenen Ausgestaltung des erfindungsgemäßen Nährbodens, mit dem neben Mutans Streptokokken auch gleichzeitig Lactobazillen bestimmt werden können, als weiterer Nährboden ein Nährboden auf der Basis eines Rogosa Agars ausgewählt, wobei ein derartige Nährboden dann einen chemischen Aufbau besitzt, wie dieser eingangs beschrieben ist.

Bei einer besonders vorteilhaften Weiterbildung der zuvor beschriebenen Ausführungsform des erfindungsgemäßen Nährbodens weist der weitere Nährboden neben dem herkömmlichen Rogosa Agar oder dem Nährboden zum Nachweis von Hefepilzen ferner noch mindestens einen Zucker auf, wobei vorzugsweise dieser mindestens eine Zucker dann Glucose, Saccharose und/oder ein Glucose- bzw. Saccharose-Derivat ist. Insbesondere dann, wenn der weitere Nährboden den Rogosa Agar oder den Nährböden zum Nachweis von Hefepilzen und den mindestens einen Zucker in einem Massenverhältnis zwischen 1:0,2 und 1:6 enthält, weist ein derartig modifizierter weiterer Nährboden eine hohe Selektivität und Ausbeute für Lactobazillen bzw. Hefepilze auf, so daß diese Ausführungsform eine genaue und reproduzierbare qualitative und quantitative Bestimmung von Lactobazillen bzw. Hefepilzen im Speichel und/oder im Plaque erlaubt.

Ist bei der zuvor beschriebenen Ausführungsform die eine Seite des inerten Trägers mit dem Nährboden zur Bestimmung der Mutans Streptokokken und die andere Seite des inerten Trägers mit dem Nährboden zum Nachweis von Hefepilzen im Speichel und/oder im Plaque versehen, so erlaubt diese Ausführungsform des erfindungsgemäßen Nährbodens insbesondere bei Kindern unter vier Jahren, vorzugsweise aber in der Altersklasse der unter Dreijährigen, eine besonders einfache und gleichzeitige Aussage dahingehend, ob im Speichel oder im Plaque dieser Kinder neben Mutans Streptokokken auch Hefepilze vorhanden sind. Hier hat sich insbesondere bei Kindern mit massivem Kariesbefall, insbesondere bei dem Vorhandensein von Flaschen-Tee-Karies (nursing bottle syndrom), gezeigt, daß Hefepilze wichtige Indikatorkeime darstellen. Vorzugsweise wird dabei ein Nährboden zum Nachweis von Hefepilzen verwendet, der die nachfolgend wiedergegebene Zusammensetzung aufweist:

| | |
|---|---|
| Chromopeptone | 8 g bis 12 g |
| Glukose | 15 g bis 25 g |
| Chloramphenicol | 0,3 g bis 0,7 g |
| Farbstoffmischung (chromogen mix) | 1,5 g bis 2,5 g |
| Agar | 10 g bis 20 g |

Besonders geeignet zum Nachweis von Hefepilzen ist der unter der Handelsbezeichnung vertriebene Agar "BL CHROMagar Candida" (Hersteller: Becton Dickinson GmbH, Heidelberg).

Zur Herstellung eines anwendungsfertigen Nährbodens zum Nachweis von Hefepilzen werden die zuvor aufgeführten Bestandteile in einem Liter entionisiertem Wasser aufgenommen, so daß ein gelartiger Nährboden entsteht, der, falls erwünscht, mit weiteren Mengen an Zucker in den beschriebenen Konzentrationsbereichen versetzt werden kann.

Um die Anwendung des auf der einen Seite mit dem erfindungsgemäßen Nährboden und auf der anderen Seite mit dem weiteren Nährboden beschichteten inerten Trägers zu erleichtern, sieht eine weitere Ausgestaltung des erfindungsgemäßen Nährbodens vor, daß der weitere Nährboden eine gelartige Konsistenz besitzt, wobei das Gel zwischen 70 g und 250 g des weiteren Nährbodens und zwischen 930 g und 750 g Wasser enthält.

Eine besonders geeignete und vorteilhafterweise in jeder Zahnarztpraxis anwendbare Weiterbildung der zuvor beschriebenen Ausführungsform des erfindungsgemäßen Nährbodens sieht vor, daß der mit dem gelartigen Nährboden auf der Basis des mit dem mindestens einen Zucker und mit dem Peptidantibiotika-Komplex, vorzugsweise mit dem Bacitracin, versetzte Agar, insbesondere der Mitis Salivarius Agar, auf der einen Seite und mit dem gelartigen weiteren Nährboden auf der Basis des zuvor beschriebenen Rogosa Agars oder dem Nährboden zum Nachweis von Hefepilzen auf der anderen Seite beschichtete inerte Träger in einem gasdicht verschließbaren Aufbewahrungsgefäß angeordnet ist. Hierbei dient das gasdicht verschließbare Aufbewahrungsgefäß einerseits zur sterilen Aufbewahrung und Transport dieser Ausführungsform des erfindungsgemäßen Nährbodens und stellt gleichzeitig sicher, daß nach erfolgter Beaufschlagung der beiden Nährböden mit der entsprechenden Speichel- bzw. Plaqueprobe die so behandelten Nährböden in dem Aufbewahrungsgefäß bebrütet werden können, wobei insbesondere gleichzeitig während der Bebrütung in dem Aufbewahrungsgefäß eine CO₂-abspaltende Tablette, so insbesondere ein Alkalicarbonat- bzw. Alkalihydrogencarbonat-Tablette, angeordnet werden kann. Hierbei bewirkt diese CO₂-abspaltende Tablette, daß während der Bebrütung in dem gasdicht verschließbaren Aufbewahrungsgefäß eine entsprechende CO₂-Atmosphäre eingestellt wird, wodurch einerseits die Selektivität des erfindungsgemäßen Nährbodens für die Mutans Streptokokken und die Lactobazillen bzw. die Hefepilze erhöht und andererseits unerwünschte Nebenreaktionen ausgeschlossen werden. Eine derartige Ausführungsform eignet sich hervorragend zur gleichzeitigen Bestimmung von Mutans Streptokokken und Lactobazillen bzw. Hefepilzen in Zahnarztpraxen, wobei diese Weiterbildung des erfindungsgemäßen Nährbodens dann eine reproduzierbare und quantifizierte Aussage bezüglich dieses Mikroorganismen erlaubt.

Die vorliegende Erfindung betrifft desweiteren ein Analysenset zur einfachen Erstellung einer Diagnostik, insbesondere zur Einschätzung des Risikos des Kariesentstehung.

Diese Aufgabe wird erfindungsgemäß durch ein Analysenset mit den kennzeichnenden Merkmalen des Patentanspruchs 20 gelöst.

Das erfindungsgemäße Analysenset weist einen auf der einen Seite eines inerten Trägers angeordneten Nährboden der vorstehend beschriebenen Art zum Nachweis von Mutans Streptokokken auf, während die andere Seite des Trägers mit dem weiteren Nährboden auf der Basis des zuvor beschriebenen Rogosa Agars zum Nachweis von Lactobazillen oder mit einem Nährboden zum Nachweis von Hefepilzen beschichtet ist.

Vorzugsweise kann in Weiterbildung des erfindungsgemäßen Analysensets dieses mindestens eine mit einer Säure, insbesondere mit einer 0,005 M (n) Salzsäure, gefüllte Carpule sowie mindestens eine Kanüle umfassen.

Das erfindungsgemäße Analysenset weist eine Reihe von Vorteilen auf. So ist zunächst festzuhalten, daß das zuvor beschriebene erfindungsgemäße Analysenset eine exakte qualitative und quantitative sowie gleichzeitige Bestimmung von Mutans Streptokokken und von Lactobazillen bzw. von Hefepilzen im Speichel und/oder im Plaque erlaubt. Zusätzlich hierzu kann die zuvor angesprochene Weiterbildung des erfindungsgemäßen Analysensets noch eine Bestimmung der Pufferkapazität des Speichels ermöglichen. Dies wird dadurch erreicht, daß in die mit einer Säure, insbesondere mit einer 0,005 M (n) Salzsäure, gefüllte Carpule dann eine definierte Menge Speichel aufgezogen wird, so daß nach Vermischen des Speichels mit der Säure der pH-Wert dieser flüssigen Mischung, der eine quantitative Angabe zur Pufferkapazität der Probe darstellt, einfach bestimmt werden kann.

Um das Einfüllen des Speichels in die mit Säure gefüllte Carpule zu bewirken, wird die im erfindungsgemäßen Analysenset vorhandene Carpule in eine übliche medizinische Spritze eingesetzt. Auf diese Spritze wird die in dem Analysenset enthaltene Kanüle aufgeschraubt, so daß dann über diese Spritze die Carpule durch Zurückziehen des Spritzenkolbens bis zu einer vorgegebenen Markierung oder einem entsprechenden Anschlag mit einer vorgegebenen Menge der Speichelprobe bzw. der Plaqueaufschlämmung gefüllt wird. Nach Vermischung der Probe mit der Säure erfolgt anhand des Farbumschlages dann die entsprechende Auswertung. Nach erfolgter Bestimmung der Konzentration an Mutans Streptokokken, Lactobazillen oder an Hefepilzen und ggf. der Pufferkapazität kann dann das erfindungsgemäße Analysenset einfach entsorgt werden.

Eine Weiterbildung des zuvor beschriebenen erfindungsgemäßen Analysensets sieht vor, daß das Analysenset mindestens eine CO₂-abspaltende Tablette aufweist. Hierbei dient diese CO₂-abspaltende Tablette im Wesentlichen dazu, daß nach Inkubieren des Nährbodens zum Nachweis von Mutans Streptokokken und des weiteren Nährbodens zum Nachweis von Lactobazillen bzw. zum Nachweis von Hefepilzen der mit diesen beiden Nährböden versehene inerte Träger in einem gasdicht verschlossenen Aufbewahrungsgefäß aufbewahrt wird, wobei in diesem gasdichten Aufbewahrungsgemäß gleichzeitig dann während der Bebrütung die CO₂-abspaltende Tablette angeordnet wird. Diese CO₂-abspaltende Tablette bewirkt, daß während der Bebrütung das gasdichte Aufbewahrungsgefäß mit einer entsprechenden CO₂-Atmosphäre beladen wird, so daß dementsprechend einerseits die Selektivität für den Nachweis von Mutans Streptokokken und Lactobazillen erhöht wird und andererseits unerwünschte Nebenreaktionen durch die CO₂-Atmosphäre ausgeschlossen werden.

Bei einer weiteren Ausführungsform des erfindungsgemäßen Analysensets umfaßt das erfindungsgemäße Analysenset mindestens ein Paraffinstück und/oder einen pH-Wert-Indikator. Hierbei dient dieses Paraffinstück dazu, daß der jeweilige Patient, dessen Speichel bzw. Plaque auf die Anwesenheit von Mutans Streptokokken und Lactobazillen bzw. Hefepilzen untersucht werden soll, vor Durchführung der diesbezüglichen Untersuchung dieses Paraffinstück für einen vorgegebenen Zeitraum kaut, um so den Speichelfluß anzuregen, während der pH-Wert-Indikator zur Bestimmung des pH-Wertes des Speichels verwendet wird, wie dies noch beschrieben wird.

Um bei den zuvor beschriebenen Ausführungsformen des erfindungsgemäßen Analysensets die Bestimmung der Pufferkapazität des Speichels weiter zu vereinfachen, sieht eine Weiterbildung des erfindungsgemäßen Analysensets vor, daß hierbei die Carpule neben der Säure, die insbesondere in einer vorgegebenen Menge und Konzentration in der Carpule enthalten ist, noch einen Indikator, insbesondere einen flüssigen Indikator, enthält. Hierdurch wird möglich, daß nach Einfüllen einer Speichelprobe bzw. einer Plaqueaufschlämmung in die Carpule die Pufferkapazität dieser Probe direkt anhand eines Farbumschlages abzulesen ist, wodurch die Sicherheit und Reproduzierbarkeit der Bestimmung der Pufferkapazität weiter verbessert wird.

Ebenso ist es möglich, daß das Analysenset mit einem pH-Wert-Indikator, so zum Beispiel einem flüssigen Indikator, einem festen Indikator und/oder einem Indikator-Streifen, versehen ist, so daß hierbei dann der gepufferte Speichel aus der Carpule entnommen und dessen pH-Wert bestimmt wird.

Insbesondere handelt es sich bei dem zuvor beschriebenen Indikator um einen flüssigen Universalindikator, wobei dieser flüssige Universalindikator einen pH-Anzeigebereich zwischen pH 4 und pH 10 aufweist.

Um die visuelle Ablesung der Pufferkapazität des Speichels bzw. der Plaqueaufschlämmung zu erleichtern, bietet es sich besonders vorteilhaft an, daß in dem erfindungsgemäßen Analysenset eine Farbskala enthalten ist, so daß durch visuellen Vergleich der Farbskala mit dem erzielten Farbausfall der mit dem Speichel gefüllten Carpule, die den Universalindikator sowie die zuvor beschriebene Säure enthält, die Pufferkapazität der jeweiligen Probe besonders einfach und schnell bestimmt werden kann.

Eine andere vorteilhafte Weiterbildung des erfindungsgemäßen Analysenset sieht vor, daß das Analysenset mindestens eine Maske umfaßt, die auf dem inerten Träger befestigbar ist, so daß durch diese Maske der auf der einen Seite des Trägers angeordnete Nährboden auf der Basis des modifizierten Agars, insbesondere des modifizierten Mitis Salivarius Agars, und/oder der auf der anderen Seite des Trägers vorgesehene weitere Nährboden auf der Basis des Rogosa Agars bzw. zum Nachweis von Hefepilzen in einzelne Felder unterteilbar ist. Diese Ausführungsvariante des erfindungsgemäßen Analysensets ermöglicht es somit, wahlweise einen Nährboden oder beide Nährböden in einzelne Felder zu unterteilen, so daß einzelne Mund- oder insbesondere Zahnbereiche spezifisch auf Mutans Streptokokken und Lactobazillen oder Hefepilze mit einem einzigen Test untersucht werden können. Hierzu ist es dann lediglich erforderlich, diese aus dem Mundbereich bzw. von dem speziellen Zahn entnommene Probe auf das jeweilige Feld des entsprechend unterteilten Nährbodens aufzutragen, so daß nach Bebrüten unschwer die dort herrschenden mikrobiologischen Verhältnisse bezüglich der Konzentration an Mutans Streptokokken und/oder Lactobazillen oder an Mutans Streptokokken und/oder Hefepilzen abgelesen werden können. Vorzugsweise ist diese Maske noch mit einem Beschriftungsteil versehen, so daß dementsprechend unschwer eine Zuordnung der gewonnenen Werte zu speziellen Mund- bzw. Zahnbereichen sicher erfolgen kann.

Bei einer anderen Ausführungsform des erfindungsgemäßen Analysensets weist dieses desweiteren ein kalibriertes Gefäß zur Aufnahme von Speichel auf. Hierdurch wird es möglich, daß bei der Speicheluntersuchung zusätzlich noch ein weiterer Parameter gemessen werden kann, wobei es sich bei diesem Parameter um das Speichelvolumen handelt, das der Patient innerhalb einer vorgegebenen Zeit beim Kauen des zuvor beschriebenen Paraffinstückes erzeugt.

Eine weitere, vorteilhafte Ausgestaltung des erfindungsgemäßen Analysensets sieht vor, daß das Analysenset desweiteren eine Einmalspritze enthält. Hierbei dient diese Einmalspritze dazu, Speichel oder eine Plaqueaufschlämmung in die mit Säure, vorzugsweise in die mit 0,005 M (n) Salzsäure, und den Universalindikator gefüllte Carpule zu injizieren, wobei die Menge des injizierten Speichels wahlweise an der Einmalspritze oder besonders vorteilhaft durch eine an der Carpule vorgesehene Markierung, bis zu der der in der Carpule vorgesehenen Nachrutschstopfen axial entgegengesetzt zur Durchstechmembrane verschoben wird, festlegbar ist. Durch diese Ausführungsform des Analysensets wird sichergestellt, daß der jeweilige Anwender des erfindungsgemäßen Analysensets alle für die Bestimmung der Pufferkapazität des Speichels erforderlichen Geräte in diesem Analysenset vorfindet, so daß dementsprechend die diesbezügliche Analyse besonders einfach und schnell und somit auch besonders wirtschaftlich durchführbar ist.

Die zuvor beschriebenen Ausführungsformen des erfindungsgemäßen Nährbodens, bei dem der Nährboden bzw. der zuvor beschriebene weitere Nährboden auf einem inerten Träger jeweils auf einer Seite aufgetragen ist, läßt sich insbesondere einfach, schnell und problemlos sowie reproduzierbar zum Nachweis von Mutans Streptokokken einerseits und zum Nachweis von Lactobazillen oder von Hefepilzen andererseits verwenden. Hierbei wird bei diesem Nachweisverfahren zunächst eine Speichel- oder Plaqueprobe beim Patienten entnommen und auf den Nährboden zum Nachweis von Mutans Streptokokken sowie auf den weiteren Nährboden zum Nachweis von Lactobazillen bzw. Hefepilzen aufgetragen. Die so mit der Speichelprobe beaufschlagten Nährböden werden hiernach vorzugsweise in einem gasdicht zu verschließenden Behältnis, das ggf. zuvor mit einer CO₂-Atmosphäre gefüllt wurde, in einen geeigneten Brutschrank überführt, so daß das diesbezügliche Aufbewahrungsbehältnis dann dort für eine vorgegebene Bebrütungszeit, insbesondere zwischen 24 und 48 Stunden, bei 37 °C ± 4 °C bebrütet wird.

Nach Ablauf dieser Bebrütungszeit erfolgt dann eine visuelle Auswertung und Quantifizierung der auf der einen Seite des inerten Trägers auf dem ersten Nährboden angezüchteten Mutans Streptokokken sowie der auf der anderen Seite des inerten Trägers auf dem dort angeordneten weiteren Nährboden angezüchteten Lactobazillen bzw. Hefepilze. Durch visuellen Vergleich mit entsprechenden Standards kann dann die Konzentration an Mutans Streptokokken und Lactobazillen bzw. an Mutans Streptokokken und Hefepilzen quantifiziert werden, wie dies in der Mikrobiologie üblich ist.

Vorteilhafte Weiterbildungen des erfindungsgemäßen Nährbodens sowie des Analysensets sind in den Unteransprüchen angegeben.

Der erfindungsgemäße Nährboden sowie das erfindungsgemäße Analysenset werden nachfolgend anhand eines Ausführungsbeispiels in Verbindung mit den Zeichnungen näher erläutert. Es zeigen:
- Figur 1: eine schematische, teilweise perspektivische Ausführungsform des Analysensets;
- Figur 2: der in dem Analysenset gemäß Figur 1 enthaltene und auf einem inerten Träger angeordnete Nährboden in einem Schnitt längs der Linie A-B in Figur 1;
- Figur 3: eine vergrößerte Darstellung der in Figur 1 gezeigten Maske in der Draufsicht;
- Figur 4: eine mit Figur 2 vergleichbare Darstellung des auf einen Träger angeordneten Nährbodens, jedoch mit der in Figur 3 gezeigten aufgesetzten Maske; und
- Figur 5: eine Schnittansicht durch eine Carpule.

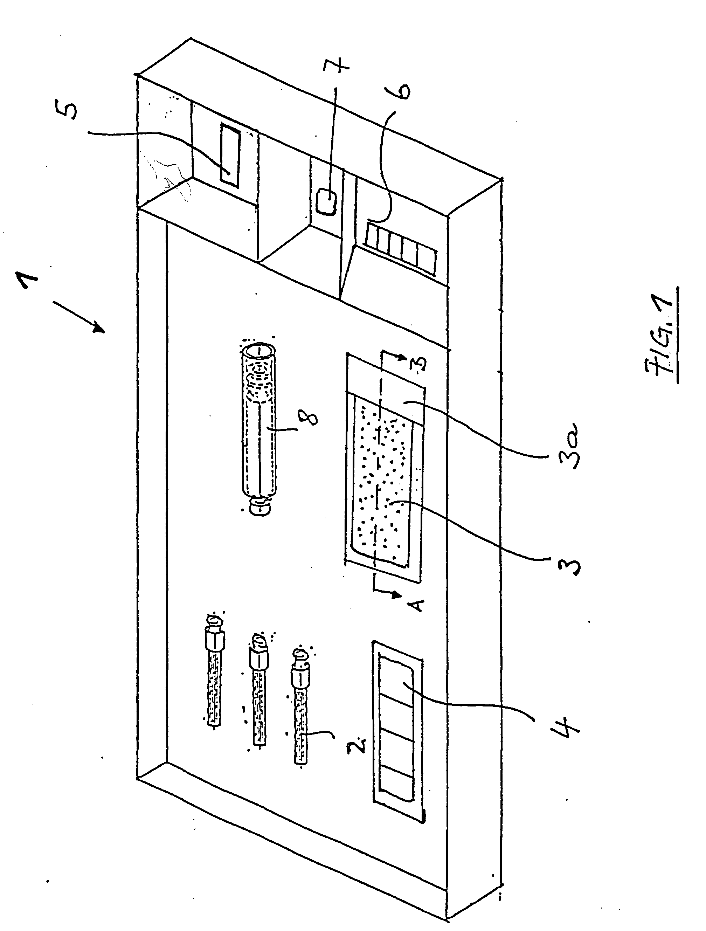

In den Figuren 1 bis 5 sind die selben Teile mit den selben Bezugsziffern versehen.

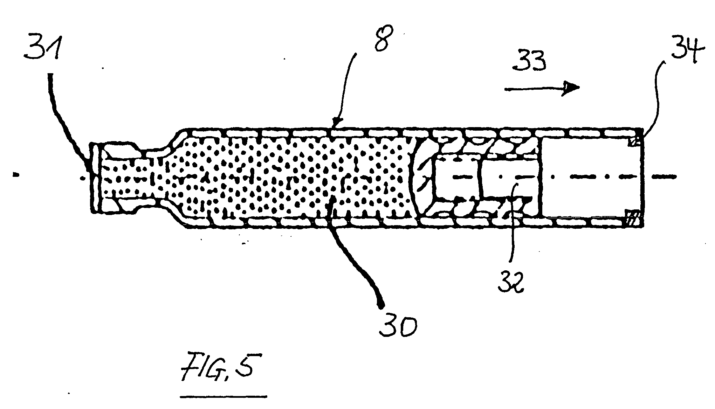

Wie der Figur 1 zu entnehmen ist, weist das Analysenset 1 zunächst eine entsprechende Anzahl von Kanülen 2 auf, wobei bei der in Figur 1 gezeigten Ausführungsform beispielhaft drei Kanülen abgebildet sind. Ferner umfaßt das Analysenset 1 eine der Anzahl der Kanülen 2 entsprechende Anzahl von Carpulen 8, wobei in Figur 1 nur beispielhaft eine einzige Carpule 8 abgebildet ist. Der nähere Aufbau dieser Carpule 8 wird anhand der Figur 5 nachfolgend noch erläutert.

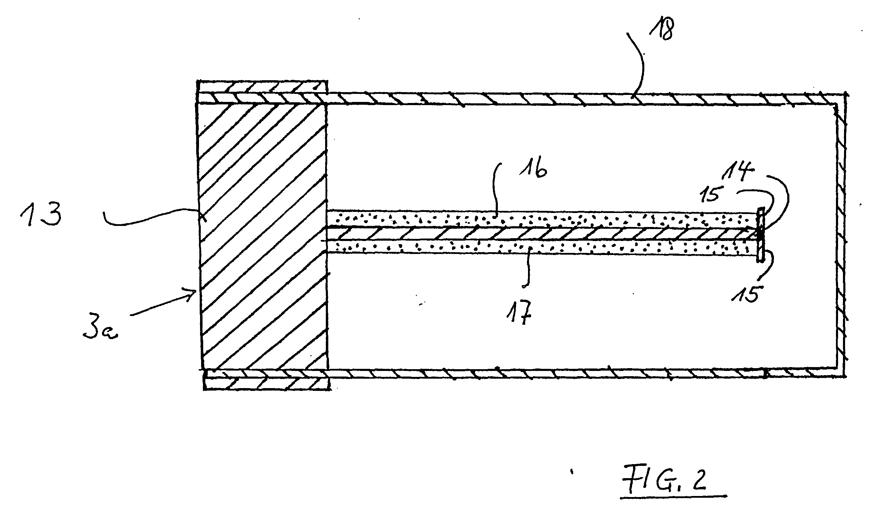

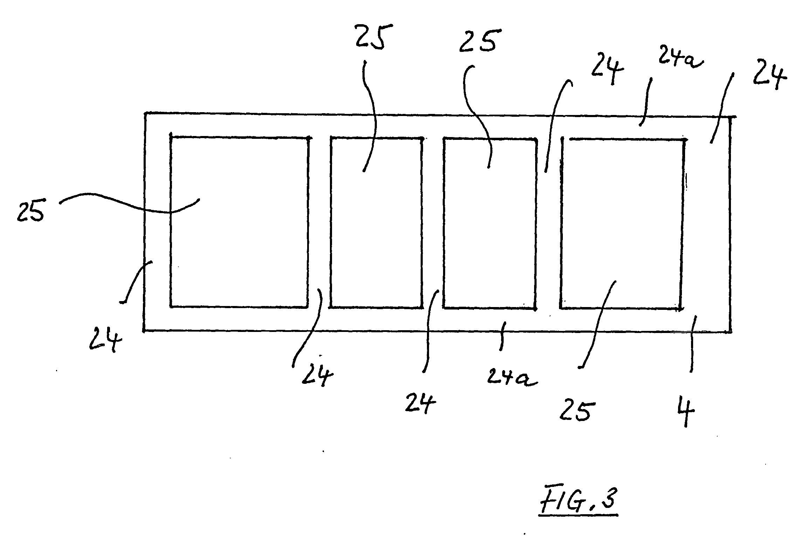

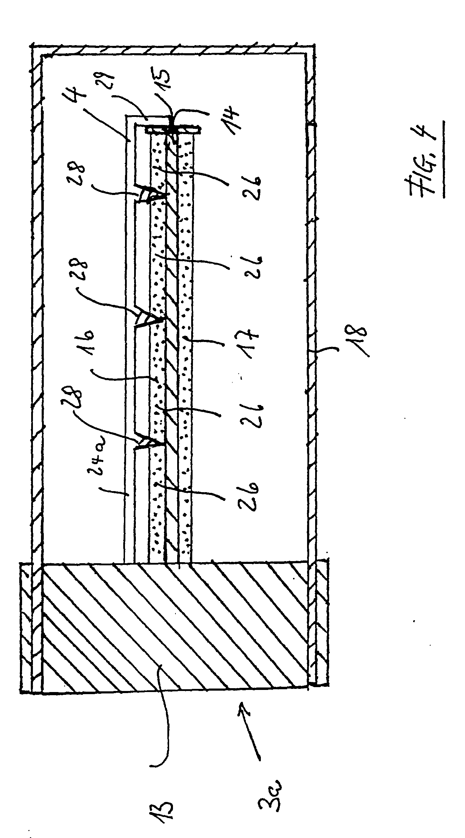

Neben der Carpule 8 und den Kanülen 2 sind in dem Analysenset 1 desweiteren eine entsprechende Anzahl mit Nährböden versehene inerte Kunststoffträger 3 a vorhanden, wobei diese mit Nährböden 3 versehene Kunststoffträger 3 a nachfolgend noch anhand der Figuren 2 und 4 näher erläutert werden. Auch weist das Analysenset eine schematisch in Figur 1 gezeigte Maske 4 auf, wobei die Maske 4 nachfolgend noch anhand der Figur 3 im Detail erläutert wird. In dem Analysenset 1 ist desweiteren eine der Anzahl der Kanülen 2 entsprechende Anzahl von Paraffinblöcken 5 und eine der Kunststoffträger 3 a entsprechende Anzahl von CO₂-abspaltenden Tabletten 7 vorgesehen, wobei jeweils in der Figur 1 nur ein Paraffinblock 5 und eine CO₂-abspaltende Tablette 7 beispielhaft gezeigt ist. Darüber hinaus weist das Analysenset 1 noch eine Farbskala 6 auf.

Wie am besten in den Schnittansichten der Figuren 2 und 4 zu erkennen ist, weist jeder inerte Kunststoffträger 3 a einen Griffabschnitt 13 auf, wobei sich an den Griffabschnitt 13 beidseitig ein schalenförmiger Bereich 14 anschließt. Hierbei umfaßt der schalenförmige Bereich 14 stegartige Seitenwandabschnitte 15, wobei in den Figuren 2 und 4 nur die stirnseitigen Stegabschnitte 15 abgebildet sind.

Die schalenförmigen Bereiche 14 dienen zur Aufnahme des Nährbodens 3, der in den Figuren 2 und 4 mit 16 bzw. 17 beziffert ist, wobei einer der beiden Nährböden 16 bzw. 17 das zuvor beschriebene modifizierte Mitis Salivarius Agar und der andere Nährboden 17 bzw. 16 das ebenfalls vorstehend beschriebene Rogosa Agar oder einen Nährboden zum Nachweis von Hefepilzen darstellen. Der inerte Kunststoffträger 3 a ist innerhalb eines gasdicht verschließbaren Aufbewahrungsgefäßes 18 angeordnet, wobei das Aufbewahrungsgefäß 18 mit dem Griffabschnitt 13 verschlossen ist.

Selbstverständlich besteht die Möglichkeit, den inerten Kunststoffträger 3 a so auszubilden, daß ein innenliegender Griffabschnitt 13 mit dem schalenförmigen Bereich 14 verbunden ist, so daß insgesamt der Kunststoffträger 3 a in dem Aufbewahrungsgefäß 18 angeordnet wird und entsprechend mit einem Schraubdeckel verschlossen ist. Diese Ausführungsform ist jedoch nicht in den Figuren 2 und 4 abgebildet.

Bei dem auf dem schalenförmigen Bereich 14 angeordneten Nährboden auf der Basis eines modifizierten Mitis Salivarius Agars handelt es sich um einen solchen Nährboden 16, der wie folgt hergestellt wird.

Zunächst wird ein käuflicher Mitis Salivarius Agar, beispielsweise von DIFCO, ausgewählt, wobei der käufliche Mitis.Salivarius Agar die folgenden Bestandteile beinhaltet:

| | |
|---|---|
| Bacto tryptose | 20 g |
| Proteose peptone Nr. 3 | 10 g |
| Proteose peptone | 10 g |
| Bacto dextrose | 2 g |
| Bacto saccharose | 100 g |
| Kaliumhydrogenphosphat | 8 g |
| Trypan blau | 0,15 g |
| Bacto kristall violett | 0,0016 g |
| Bacto Agar | 30 g |

Eine erste Hälfte dieses trockenen Nährbodens, der nachfolgend auch als Nährboden I bezeichnet ist, wird mit 360 g Saccharose (Merck 1.07651) und 3 mg Bacitracin (Sigmar B-0125) und eine zweite Hälfte dieses trockenen Nährbodens, der nachfolgend als Nährboden II bezeichnet ist, wird mit 600 g Saccharose (Merck 1.07651) und 5 mg Bacitracin (Sigmar B-0125) versetzt und vermischt. Anschließend werden diese Nährböden I und II in entionisiertem Wasser unter Ausbildung von jeweils einem Liter anwendungsfertigen Nährböden I und II aufgelöst. Hiernach wird das Wasser zum Sieden gebracht. Anschließend erfolgt eine Sterilisation der Nährböden bei 121 °C für 15 Minuten. Nach Abkühlung werden dann die gelartigen Nährböden auf den inerten Kunststoffträger 3 a, bei dem es sich um einen schwarzen Kunststoffträger handelt, aufgebracht.

Hieraus ergibt sich für den Nährboden I ein Verhältnis von Mitis Salivarius Agar zu zugesetzter Saccharose von 1:3,996 und für den Nährboden II ein Verhältnis von Mitis Salivarius Agar zu zugesetzter Saccharose von 1:6,661.

Zur Herstellung des Nährbodens 17 geht man von einem herkömmlichen Rogosa Agar aus, wobei der diesbezügliche Rogosa Agar die Zusammensetzung aufweist, wie sie vorstehend wie folgt angegeben ist.

| | |
|---|---|
| Agar Agar | 19 g |
| Tryptone | 10 g |
| Hefeextrakt | 5 g |
| Glucose | 20 g |
| Kaliumhydrogenphosphat | 6 g |
| Ammoniumhydrogencitrat | 2 g |
| Mangan-II-Sulfat 1-Hydrat | 0,1 g |
| Eisen-II-Sulfat 7-Hydrat | 0,03 g |
| Tween 80 | 1 g |

Dieser Rogosa Agar wird in 1 l Wasser aufgelöst, nachdem zuvor ein Zusatz von 1,32 ml Eisessig erfolgte. Nach Lösen des Rogosa Agars entstehen ein Liter einer gelartigen bis pastösen Zusammensetzung, die auf den schalenartigen Bereich 14 des inerten Kunststoffträgers 3 a aufgebracht wird und daran haftet.

Die Figur 3 bildet vergrößert und schematisch eine Maske 4 ab, wobei die Maske 4 derart gestaltet ist, daß zwischen den Stegen rechteckige Freiflächen 25 ausgebildet werden, wobei in der Figur 3 beispielhaft vier derartige Aussparungen 25 vorgesehen sind.

Auf seiner Unterseite besitzen die Stege, die sich quer zu den Längsstegen 24 a erstrecken, keilförmige Schneiden 28 (Figur 4), wobei diese keilförmigen Schneiden 28 eine Aufteilung des Nährbodens in Felder 26 bewirkt, wie dies in Figur 4 für den Nährboden 16 gezeigt ist. Um die Maske 4 an dem inerten Kunststoffträger 3 a zu haltern, weist die Maske 4 entsprechende Klemmabschnitte 29 auf, die in Eingriff mit den stegartigen Seitenwandabschnitten 15 bringbar sind.

In Figur 5 ist schematisch eine Carpule 8 vergrößert abgebildet, wobei die Carpule 8 kopfseitig eine Durchstechmembrane 31 und fußseitig einen Kolben 32 aufweist, wobei der Kolben 32 axial in Pfeilrichtung 33 und umgekehrt hierzu verschiebbar ist (Figur 5). Im Innenraum 30 der Carpule 8 ist eine 0,005 M (n) Salzsäurelösung sowie eine Lösung eines Universalindikators angeordnet, wobei das Volumen dieser beiden Lösungen insgesamt 1,27 ml beträgt. Fußseitig an der Carpule 8 ist ein Begrenzungselement 34 vorgesehen, derart, daß die axiale Verschiebung des Kolbens 32 in Pfeilrichtung 33 durch dieses Begrenzungselement 34 begrenzt wird. Hierdurch wird sichergestellt, daß bei der Verschiebung des Kolbens 32 in Pfeilrichtung 33 in den Innenraum 30 der Carpule 8 maximal ein Gesamtvolumen von 1,7 ml Flüssigkeit eingezogen werden kann.

Will nunmehr der jeweilige Anwender das zuvor beschriebene und in den Figuren 1 bis 5 gezeigte Analysenset verwenden, so muß der jeweilige Patient zunächst für eine vorgegebene Zeit (in der Regel fünf Minuten) den aus dem Analysenset 1 entnommenen Paraffinblock 5 kauen. Hierdurch wird der Speichelfluß beim Patienten angeregt, so daß die ausgespuckte Speichelmenge dann über einen entsprechenden Meßzylinder erfaßt und gemessen werden kann.

Zur Bestimmung der Pufferkapazität des Speichels entnimmt der Benutzers des Analysensets 1 dem Analysenset 1 eine Carpule 8, wobei die Carpule 8 mit der Mischung aus Salzsäure und Universalindikator (Meßbereich pH 4 bis 10) gefüllt ist. Diese Carpule 8 wird dann in eine herkömmliche Spritze eingesetzt, wobei die Spritze mit einer aus dem Analysenset 1 entnommenen Kanüle 2 bestückt wird. In die in die Spritze eingesetzte Carpule wird dann durch eine entsprechende Bewegung des Spritzenkolbens, der in Eingriff mit den innerhalb der Carpule 8 angeordneten Kolben 32 (Figur 5) tritt, eine vorgegebene Menge Speichel eingezogen, wobei das Volumen des Speichels ein Viertel des Volumens des Carpuleninhalts entspricht. Die axiale Verschiebung des Verschiebekolbens 32 wird dann durch den Anschlag 34 begrenzt, so daß sichergestellt ist, daß stets die gleiche Menge Speichel in die Carpule 8 eingezogen wird.

Nunmehr wird die zusätzlich mit dem Speichel gefüllte Carpule 8 der Spritze entnommen und die auf der Spritze noch arretierte Einmalkanüle, die eine Länge von 32 mm aufweist und einen Durchmesser von 0,9 mm besitzt, abgenommen und verworfen.

Die aus der Spritze entnommene Carpule 8, bei der es sich um eine durchsichtige Glascarpule handelt, erlaubt dann unmittelbar eine visuelle Bewertung der Farbänderung des Carpuleninhalts, wobei eine Quantifizierung dieser Farbänderung durch den visuellen Vergleich mit der in dem Analysenset 1 enthaltenen Farbskala 6 erfolgt. Hieraus bestimmt der jeweilige Anwender dann die Pufferkapazität des Speichels.

Eine weitere Speichelprobe wird dann auf den Nährboden 16 und eine andere Speichelprobe auf den Nährboden 17 (Figur 2) aufgebracht, wobei nach Auftragen der Speichelproben der mit den Nährböden 16 und 17 beschichtete Kunststoffträger 3 a in das Aufbewahrungsgefäß 18 eingebracht wird. Zuvor ist eine CO₂-abspaltende Tablette 7 aus dem Analysenset 1 entnommen und in das Aufbewahrungsgefäß 18 eingebracht worden.

Nachdem das Aufbewahrungsgefäß 18 gasdicht verschlossen ist, wird das Aufbewahrungsgefäß 18 zusammen mit den darin angeordneten und mit Speichel inkubierten Nährböden 16 und 17 in einen geeigneten Brutschrank überführt, wobei das diesbezügliche Aufbewahrungsgefäß dann dort beispielsweise 24 oder 48 Stunden bei 37 °C bebrütet wird.

Nach Ablauf dieser Bebrütungszeit erfolgt dann die visuelle Auswertung und Quantifizierung der Nährböden 16 bzw. 17, wobei die angezüchteten Mutans Streptokokken sowie die auf dem weiteren Nährboden angezüchteten Lactobazillen deutlich erkennbar sind, da sie sich farblich vom Untergrund abheben. Durch visuellen Vergleich mit entsprechenden Standards kann dann die Konzentration an Mutans Streptokokken und Lactobazillen ermittelt werden, wie dies in der Mikrobiologie üblich ist.

Will hingegen der jeweilige Anwender einzelne bestimmte Mundbereiche oder einzelne Zähne gezielt auf die dort anwesenden Mikroorganismen untersuchen, so ordnet er eine oder zwei Masken 4, die ebenfalls in dem Analysenset 1 vorhanden sind, auf dem Nährboden 16 und/oder auf dem Nährboden 17 an, wie dies für den Nährboden 16 in Figur 4 gezeigt ist. Hierdurch entstehen auf dem jeweiligen Nährboden, beispielsweise dem Nährboden 16 in Figur 4, einzelne Felder 26, die dann gezielt mit den lokal entnommenen Speichelproben bzw. Plaqueaufschlämmungen inkubiert werden. Hiernach erfolgt die Weiterverarbeitung in der Weise, wie dies vorstehend beschrieben ist.

Der wesentliche Vorteil dieser Arbeitsweise ist darin zu sehen, daß in dem selben Analysengang gleichzeitig alle lokal entnommenen Proben untersucht werden, so daß dementsprechend ein methodischer Fehler ausgeschlossen wird. Dies trägt wesentlich zur Steigerung der Genauigkeit bei.

Zum Nachweis, daß der mit Zucker in unterschiedlichen Konzentrationen zusätzlich versetzte und vorstehend beschriebene Mitis Salivarius Agar (Nährböden I und II) deutlich einem herkömmlichen Mitis Salivarius Agar überlegen ist, wurde eine umfangreiche klinische Studie an 55 Kindern und 135 Speichelisolaten durchgeführt. Hierbei wurde der herkömmliche Mitis Salivarius Agar mit den mit Zucker versetzten Nährböden I und II verglichen, wobei der mit Zucker versetzte Mitis Salivarius Agar den Aufbau besitzt, wie dieser vorstehend für die Nährböden I und II beschrieben ist.

Im Rahmen dieser Studie konnte festgestellt werden, daß die Keimausbeute an Mutans Streptokokken bei den mit Zucker versetzten Nährböden I und II deutlich und signifikant höher lag als die Keimausbeute an Mutans Streptokokken beim herkömmlichen Mitis Salivarius Agar. Der Nährboden II zeigte im Vergleich zum Nährboden I eine noch höhere Keimausbeute an Mutans Streptokokken.

## Patentansprüche

1. Nährboden zum Nachweis von Mutans Streptokokken im Speichel und/oder im Plaque, wobei der Nährboden neben Agar zusätzlich noch mindestens einen Zucker enthält, **dadurch gekennzeichnet**, daß das Massenverhältnis des Agars zu dem mindestens einen Zucker zwischen 1:3,89 und 1:12 variiert.

2. Nährboden nach Anspruch 1, **dadurch gekennzeichnet**, daß bei dem Nährboden das Massenverhältnis zwischen dem Agar und dem mindestens einen Zucker zwischen 1:3,89 und 1:4,25 liegt.

3. Nährboden nach Anspruch 1, **dadurch gekennzeichnet**, daß bei dem Nährboden das Massenverhältnis zwischen dem Agar und dem mindestens einen Zucker zwischen 1:5,1 und 1:10 liegt.

4. Nährboden nach einem der vorangehenden Ansprüche, **dadurch gekennzeichnet,** daß der Nährboden als Zucker mindestens ein Disaccharid aufweist.

5. Nährboden nach Anspruch 4, **dadurch gekennzeichnet**, daß der Nährboden als Disaccharid Saccharose und/oder ein Saccharose-Derivat enthält.

6. Nährboden nach einem der vorangehenden Ansprüche, **dadurch gekennzeichnet,** daß der Nährboden zwischen 0,001 Gew.% und 0,0005 Gew.% eines Peptidantibiotika-Komplexes enthält.

7. Nährboden nach Anspruch 6, **dadurch gekennzeichnet**, daß der Peptidantibiotika-Komplex Bacitracin ist.

8. Nährboden nach einem der vorangehenden Ansprüche, **dadurch gekennzeichnet,** daß der Agar ein Mitis Salivarius Agar ist.

9. Nährboden nach einem der vorangehenden Ansprüche, **dadurch gekennzeichnet,** daß der Nährboden einseitig auf einen inerten Träger aufgetragen ist.

10. Nährboden nach einem der vorangehenden Ansprüche, **dadurch gekennzeichnet,** daß der Nährboden eine gelartige Konsistenz aufweist, wobei zur Erstellung der gelartigen Konsistenz der Nährboden mit Wasser versetzbar ist.

11. Nährboden nach Anspruch 10, **dadurch gekennzeichnet**, daß der Nährboden zwischen 70 g und 150 g Agar, zwischen 272,3 g und 1.800 g des mindestens einen Zuckers und so viel Wasser, um die erforderliche gelartige Konsistenz zu erstellen, umfaßt.

12. Nährboden nach Anspruch 11, **dadurch gekennzeichnet**, daß der gelartige Nährboden zwischen 1,4 mg und 12 mg des Peptidantibiotika-Komplexes beinhaltet.

13. Nährboden nach Anspruch 12, **dadurch gekennzeichnet**, daß der Peptidantibiotika-Komplex Bacitracin ist.

14. Nährboden nach einem der vorangehenden Ansprüche, **dadurch gekennzeichnet,** daß der Nährboden einseitig auf einen inerten Träger, insbesondere auf einen Kunststoffträger, aufgetragen ist und daß die andere Seite des inerten Trägers mit einem weiteren Nährboden zum Nachweis von Lactobazillen im Speichel und/oder im Plaque oder mit einem weiteren Nährboden zum Nachweis von Hefepilzen im Speichel und/oder im Plaque versehen ist.

15. Nährboden nach Anspruch 14, **dadurch gekennzeichnet**, daß der weitere Nährboden ein Nährboden auf der Basis eines Rogosa Agars zum Nachweis von Lactobazillen ist.

16. Nährboden nach Anspruch 15, **dadurch gekennzeichnet**, daß der weitere Nährboden desweiteren noch mindestens einen Zucker aufweist.

17. Nährboden nach Anspruch 16, **dadurch gekennzeichnet**, daß der weitere Nährboden den Agar und den Zucker in einem Massenverhältnis zwischen 1:0,2 und 1:6 aufweist.

18. Nährboden nach einem der Ansprüche 14 bis 17, **dadurch gekennzeichnet**, daß der weitere Nährboden eine gelartige Konsistenz aufweist, wobei das Gel zwischen 70 g und 250 g des weiteren Nährbodens und zwischen 930 g und 750 g Wasser enthält.

19. Nährboden nach einem der vorangehenden Ansprüche, **dadurch gekennzeichnet,** daß der mit dem gelartigen Nährboden auf der einen Seite und mit dem gelartigen weiteren Nährboden auf der anderen Seite beschichtete inerte Träger in einem gasdicht verschließbaren Aufbewahrungsgefäß angeordnet ist.

20. Analysenset mit einem auf einem inerten Träger angeordneten Nährboden nach einem der vorangehenden Ansprüche, **dadurch gekennzeichnet,** daß das Analysenset (1) mindestens einen inerten Träger (3a), der auf seiner einen Seite mit dem Nährboden (3, 16, 17) und auf der anderen Seite mit dem weiteren Nährboden (17, 16) beschichtet ist, umfaßt.

21. Analysenset nach Anspruch 20, **dadurch gekennzeichnet**, daß das Analysenset neben dem inerten Träger (3a) desweiteren mindestens eine mit einer Säure gefüllte Carpule (8) und mindestens eine Kanüle (2) umfaßt.

22. Analysenset nach Anspruch 21, **dadurch gekennzeichnet**, daß die Carpule mit einer 0,005 M (n) Salzsäure gefüllt ist.

23. Analysenset nach einem der Ansprüche 20 bis 22, **dadurch gekennzeichnet**, daß das Analysenset mindestens eine CO₂-abspaltende Tablette (7) aufweist.

24. Analysenset nach einem der Ansprüche 20 bis 23, **dadurch gekennzeichnet**, daß das Analysenset (1) mindestens ein Paraffinstück (5) und/oder einen pH-Wert-Indikator umfaßt.

25. Analysenset nach einem der Ansprüche 20 bis 24, **dadurch gekennzeichnet**, daß die Carpule (8) neben der Säure noch mit einem Indikator gefüllt ist.

26. Analysenset nach Anspruch 25, **dadurch gekennzeichnet**, daß der Indikator ein flüssiger Indikator ist.

27. Analysenset nach Anspruch 26, **dadurch gekennzeichnet**, daß der Indikator ein flüssiger Universalindikator mit einem pH-Anzeigebereich zwischen pH 4 und pH 10 ist.

28. Analysenset nach einem der Ansprüche 20 bis 27, **dadurch gekennzeichnet**, daß das Analysenset (1) eine Farbskala (6) zur Ablesung des pH-Wertes der mit dem Indikator gefüllten Carpule (8) umfaßt.

29. Analysenset nach Anspruch 20 bis 28, **dadurch gekennzeichnet,** daß das Analysenset mindestens eine Maske (4) umfaßt, die auf dem inerten Träger (3a) befestigbar ist, so daß der Nährboden (16; 17) und/oder der weitere Nährboden (17; 16) in einzelne Felder (26) unterteilbar ist.

30. Analysenset nach einem der Ansprüche 20 bis 29, **dadurch gekennzeichnet**, daß das Analysenset (1) desweiteren ein kalibriertes Gefäß zur Aufnahme von Speichel aufweist.

31. Analysenset nach einem der Ansprüche 20 bis 30, **dadurch gekennzeichnet**, daß das Analysenset (1) mindestens eine Einmalspritze enthält.

## Claims

1. A culture medium for the detection of streptococcus mutans in the saliva and/or in the plaque, whereby the culture medium contains at least one sugar in addition to an agar, **characterised in that** the mass ratio of said agar to said at least one sugar varies between 1:3,89 and 1:12.

2. The culture medium according to claim 1, **characterised in that** the culture medium has a mass ratio of said agar to said at least one sugar of between 1:3,89 and 1:4,25.

3. The culture medium according to claim 1, **characterised in that** the culture medium has a mass ratio of said agar to said at least one sugar of between 1:5,1 and 1:10.

4. The culture medium according to one of the preceding claims, **characterised in that** the culture medium comprises as sugar at least one disaccharide.

5. The culture medium according to one of the preceding claims, **characterised in that** the culture medium comprises as disaccharide sucrose and/or a sucrose derivative.

6. The culture medium according to one of the preceding claims, **characterised in that** the culture medium contains between 0,001 % by weight and 0,0005 % by weight of a peptide antibiotic complex.

7. The culture medium according to claim 6, **characterised in that** said peptide antibiotic complex is bacitracin.

8. The culture medium according to one of the preceding claims, **characterised in that** said agar is a mitis salivarius agar.

9. The culture medium according to one of the preceding claims, **characterised in that** the culture medium is applied on one side of an inert support.

10. The culture medium according to one of the preceding claims, **characterised in that** the culture medium has gel-like consistency, whereby water is added to the culture medium in order to produce the gel-like consistency.

11. The culture medium according to claim 10, **characterised in that** the culture medium comprises between 70 g and 150 g of said agar between 272,3 g and 1.800 g of said at least one sugar and such amount of water to create the wanted gel-like consistency.

12. The culture medium according to claim 11, **characterised in that** the gel-like culture medium comprises between 1,4 mg and 12 mg of said peptide antibiotic complex.

13. The culture medium according to claim 12, **characterised in that** the peptide antibiotic complex bacitracin.

14. The culture medium according to one of the preceding claims, **characterised in that** the culture medium is applied on one side of said inert support, preferably a plastic support, and that on the other side of said inert support a further culture medium for the detection of lactobacillus in the saliva and/or in the plaque or a further culture medium for the detection of yeast fungus in the saliva and/or the plaque is applied.

15. The culture medium according to claim 14, **characterised in that** said further culture medium is based on a rogosa agar for the detection of lactobacillus.

16. The culture medium according to claim 15, **characterised in that** said further culture medium moreover comprises at least one sugar.

17. The culture medium according to claim 16, **characterised in that** said further culture medium contains the agar and the sugar in mass ratio of between 1:0,2 and 1:6.

18. The culture medium according to one of the claims 14 to 17, **characterised in that** said further culture medium has a gel-like consistency, whereby the gel contains between 70 g and 250 g of the further culture medium and between 930 g and 750 g of water.

19. The culture medium according to one of the preceding claims, **characterised in that** the inert support being coated on one side with said gel-like culture medium and on the other side with said further gel-like culture medium is arranged within a gastight lockable storage vessel.

20. An analysis set with a culture medium arranged on an inert support according to one of the preceding claims, **characterised in that** the analysis set (1) comprises at least one inert support (3 a) being coated on one side with said culture medium (3, 16, 17) and on the other side with said further culture medium (17, 16).

21. The analysis set according to claim 20, **characterised in that** the analysis set comprises, in addition to said inert support (3 a), at least one carpule (8) filled with an acid and at least one cannula (2).

22. The analysis set according to claim 21, **characterised in that** the carpule is filled with a 0,005 M(n) hydrochloric acid.

23. The analysis set according to claim 20 to 22, **characterised in that** the analysis set comprises at least one tablet (7) releasing CO₂.

24. The analysis set according to one of the claims 20 to 23, **characterised in that** the analysis set (1) comprises at least one paraffin piece (5) and/or a pH-value indicator.

25. The analysis set according to one of the claims 20 to 24, **characterised in that** the carpule (8) is filled with an indicator.

26. The analysis set according to claim 25, **characterised in that** the said indicator is a liquid indicator.

27. The analysis set according to claim 26, **characterised in that** the indicator is a liquid universal indicator with a pH-indication range between pH 4 and pH 10.

28. The analysis set according to one of the claims 20 to 27, **characterised in that** the analysis set (1) comprises a colour scale (6) to measure the pH-value of the carpule (8) being filled with the indicator.

29. The analysis set according to claim 20 to 28, **characterised in that** the analysis set comprises at least one mask (4) which can be fixed onto the inert support (3 a), so that said culture medium (16; 17) and/or said further culture medium (17; 16) can be subdivided into single sections (26).

30. The analysis set according to one of the claims 20 to 29, **characterised in that** said analysis set (1) comprises moreover a calibrated vessel for taking up the saliva.

31. The analysis set according to one of the claims 20 to 30, **characterised in that** said analysis set (1) comprises at least one disposable syringe.

## Revendications

1. Milieu de culture pour détecter des Streptocoques mutans dans la salive et/ou dans la plaque, le milieu de culture contenant, outre de l'agar, encore au moins un sucre en supplément, **caractérisé en ce que** le rapport massique entre l'agar et ledit au moins un sucre varie entre 1/3,89 et 1/12.

2. Milieu de culture suivant la revendication 1, **caractérisé en ce que**, dans le milieu de culture, le rapport massique entre l'agar et ledit au moins un sucre est compris entre 1/3,89 et 1/4,25.

3. Milieu de culture suivant la revendication 1, **caractérisé en ce que**, dans le milieu de culture, le rapport massique entre l'agar et ledit au moins un sucre est compris entre 1/5,1 et 1/10.

4. Milieu de culture suivant l'une des revendications précédentes, **caractérisé en ce que** le milieu de culture présente au moins un disaccharide comme sucre.

5. Milieu de culture suivant la revendication 4, **caractérisé en ce que** le milieu de culture contient, comme disaccharide, du saccharose et/ou un dérivé de saccharose.

6. Milieu de culture suivant l'une des revendications précédentes, **caractérisé en ce que** le milieu de culture contient entre 0,001% en poids et 0,0005% en poids d'un complexe d'antibiotiques peptidiques.

7. Milieu de culture suivant la revendication 6, **caractérisé en ce que** le complexe d'antibiotiques peptidiques est de la bacitracine.

8. Milieu de culture suivant l'une des revendications précédentes, **caractérisé en ce que** l'agar est un agar Mitis Salivarius.

9. Milieu de culture suivant l'une des revendications précédentes, **caractérisé en ce que** le milieu de culture est supporté sur un support inerte d'un côté.

10. Milieu de culture suivant l'une des revendications précédentes, **caractérisé en ce que** le milieu de culture présente une consistance de type gel, de l'eau pouvant être ajoutée au milieu de culture pour la réalisation de la consistance gélatineuse.

11. Milieu de culture suivant la revendication 10, **caractérisé en ce que** le milieu de culture comporte entre 70 g et 150 g d'agar, entre 272,3 g et 1800 g dudit au moins un sucre et de l'eau en suffisance pour réaliser la consistance gélatineuse requise.

12. Milieu de culture suivant la revendication 11, **caractérisé en ce que** le milieu de culture gélatineux contient entre 1,4 mg et 12 mg du complexe d'antiobiotiques peptidiques.

13. Milieu de culture suivant la revendication 12, **caractérisé en ce que** le complexe d'antibiotiques peptidiques est de la bacitracine.

14. Milieu de culture suivant l'une des revendications précédentes, **caractérisé en ce que** le milieu de culture est supporté sur un support inerte d'un côté, en particulier sur un support en matière synthétique, et en ce que l'autre côté du support inerte est pourvu d'un autre milieu de culture pour détecter des lactobacilles dans la salive et/ou dans la plaque ou d'un autre milieu de culture pour détecter des levures dans la salive et/ou dans la plaque.

15. Milieu de culture suivant la revendication 14, **caractérisé en ce que** l'autre milieu de culture est un milieu de culture à base d'un agar Rogosa pour détecter des lactobacilles.

16. Milieu de culture suivant le revendication 15, **caractérisé en ce que** l'autre milieu de culture présente par ailleurs encore au moins un sucre.

17. Milieu de culture suivant la revendication 16, **caractérisé en ce que** l'autre milieu de culture présente l'agar et le sucre dans un rapport massique compris entre 1/0,2 et 1/6.

18. Milieu de culture suivant l'une des revendications 14 à 17, **caractérisé en ce que** l'autre milieu de culture présente une consistance de type gel, le gel contenant entre 70 g et 250 g de l'autre milieu de culture et entre 930 g et 750 g d'eau.

19. Milieu de culture suivant l'une des revendications précédentes, **caractérisé en ce que** le support inerte enduit d'un côté par le milieu de culture gélatineux et de l'autre côté par l'autre milieu de culture gélatineux est agencé dans un récipient de conservation qui peut être obturé d'une manière étanche aux gaz.

20. Ensemble pour analyses comprenant un milieu de culture agencé sur un support inerte suivant l'une des revendications précédentes, **caractérisé en ce que** l'ensemble pour analyses (1) comporte au moins un support inerte (3a), qui est enduit sur un de ses côtés par le milieu de culture (3, 16, 17) et sur l'autre côté par l'autre milieu de culture (17,16).

21. Ensemble pour analyses suivant la revendication 20, **caractérisé en ce que** l'ensemble pour analyses comporte, outre le support inerte (3a), au moins une cartouche (8) remplie d'un acide et au moins une canule (2).

22. Ensemble pour analyses suivant la revendication 21, **caractérisé en ce que** la cartouche est remplie d'un acide chlorhydrique 0,005 M (n).

23. Ensemble pour analyses suivant l'une des revendications 20 à 22, **caractérisé en ce que** l'ensemble pour analyses présente au moins un comprimé dégageant du CO₂ (7).

24. Ensemble pour analyses suivant l'une des revendications 20 à 23, **caractérisé en ce que** l'ensemble pour analyses (1) comporte au moins un morceau de paraffine (5) et/ou un indicateur de valeur de pH.

25. Ensemble pour analyses suivant l'une des revendications 20 à 24, **caractérisé en ce que** la cartouche (8) est remplie, en plus de l'acide, d'un indicateur.

26. Ensemble pour analyse suivant la revendication 25, **caractérisé en ce que** l'indicateur est un indicateur liquide.

27. Ensemble pour analyses suivant la revendication 26, **caractérisé en ce que** l'indicateur est un indicateur universel liquide ayant une gamme d'indications de pH comprise entre pH 4 et pH 10.

28. Ensemble pour analyses suivant l'une des revendications 20 à 27, **caractérisé en ce que** l'ensemble pour analyses (1) comporte une échelle des couleurs (6) pour la lecture de la valeur du pH de la cartouche (8) remplie de l'indicateur.

29. Ensemble pour analyses suivant l'une des revendications 20 à 28, **caractérisé en ce que** l'ensemble pour analyses comporte au moins un masque (4) qui peut être fixé sur le support inerte (3a) de façon que le milieu de culture (16;17) et/ou l'autre milieu de culture (17; 16) puissent être subdivisés en zones individuelles (26).

30. Ensemble pour analyses suivant l'une des revendications 20 à 29, **caractérisé en ce que** l'ensemble pour analyses (1) présente, en outre, un récipient calibré pour recevoir de la salive.

31. Ensemble pour analyses suivant l'une des revendications 20 à 30, **caractérisé en ce que** l'ensemble pour analyses (1) contient au moins une seringue à usage unique.
